# EUROPEAN PATENT SPECIFICATION

(11) **EP 4 158 590 B1**
(45) Date of publication and mention of the grant of the patent: **29.10.2025**
(21) Application number: 21727563.5
(22) Date of filing: 11.05.2021
(51) Int. Cl.: G06T 7/00, C14B 17/00, G01N 33/44

(54) **METHOD AND APPARATUS FOR IDENTIFYING POSSIBLE SURFACE DEFECTS OF A LEATHER HIDE**
VERFAHREN UND VORRICHTUNG ZUR ERKENNUNG MÖGLICHER OBERFLÄCHENFEHLER EINER LEDERHAUT
PROCÉDÉ ET APPAREIL POUR IDENTIFIER LES DÉFAUTS DE SURFACE POSSIBLES D'UNE PEAU DE CUIR

(30) Priority: 29.05.2020 IT 202000012928
(43) Date of publication of application: 05.04.2023
(73) Proprietor: Conceria Pasubio S.p.A., 36071 Arzignano (Vicenza) (IT)
(72) Inventor: PRETTO, Luca, 36071 Arzignano (Vicenza) (IT)
(74) Representative: Sandri, Sandro
(86) International application number: PCT/IB2021/053994
(87) International publication number: WO 2021/240279

(56) References cited:
- WO-A1-2010/130226
- WO-A1-2011/030360
- DE-A1- 4 216 469
- US-A1- 2001 012 381
- US-A1- 2008 316 476
- US-A1- 2014 368 634
- US-A1- 2019 360 992

## Description

### Technical field

The present invention relates to a method for identifying surface defects of a leather hide.

The invention further relates to an apparatus for identifying surface defects of a leather hide configured to implement the aforesaid method.

### Prior art

In the tanning industry, a major problem to be managed is represented by the damage that the hide of the animal undergoes both in the course of its lifetime and during processing for tanning (and tanning itself).

The nature, quantity and extent of the defects present on each hide influence the final value thereof, since the defective surface portions must be discarded or used only marginally.

In the first steps of the tanning process, the activity that intercepts and classifies defects is the so-called "wet selection". This step is of crucial importance, being decisive for the final result of the article it is intended to produce.

At present, the expert operators in the tanning industry carry out the selection operation by visually inspecting each leather hide, in order to detect the surface defects and evaluate the usable defect-free space in order also to define the suitable category in which to classify the hide examined.

Based on their origin, the defects of leather hides can be divided into four macro categories: pre-existing defects on the animal; defects deriving from an incorrect slaughter and skinning; defects due to poor preservation of the hides; and defects due to an imprecise production process.

This procedure, being performed in a totally manual manner, poses clear limits in the location and identification of defects, which are attributable to the need to: train and/or rely on expert personnel; spend a significant amount of time evaluating each defect; and evaluate each defect with subjective parameters that differ for each expert operator.

In fact, even with the use of trained personnel it is unthinkable to improve (and homogenise) in any considerable way the results obtained by applying the manual inspection method described above. The manual inspection process, in fact, is largely subjective as it is influenced by the experience acquired by each operator. Moreover, the repetitiveness of this process increases the likelihood that an operator will classify some defects of the hide analysed in a non-homogeneous or incorrect manner, for example not judging them to be defects and thus not allowing that surface portion of the leather hide to be discarded.

In this regard, the Applicant has conducted an internal study in order to evaluate the agreement between the reference standard and the result of the identification and classification of surface defects carried out by operators specialised as per the prior art. The aim of the study was to evaluate the agreement of specialised operators with themselves, with other specialised operators and with the reference standard. For the purpose of conducting the study, fifty leather hides were used as a sample to which a standard category of reference was assigned and, furthermore, each category had the same number of samples. Four pairs of specialised operators participated in the study; they evaluated the samples in random order and rated them at least twice three days apart, rating them the same total number of times. The study showed that among the specialised operators there is moderate agreement with themselves, whereas the agreement between them and with the reference standard tends to be low.

Document DE 4216469 A1 discloses a device for classifying defects in hides to be processed.

In particular, the pressed hide is made to pass at a predefined speed through an illumination device having a detection line for linear recording. In this manner it is possible to obtain images of the profile of the grain side of the hide and/or of the intensity of the light transmitted linearly from the back side of the hide. The information obtained from the intensity of the detected radiation can be processed in parallel in an evaluation device (30) in which the expert information on the types of defects can be converted into information of classification according to the size, type, position and amount of the defects in the hide in order to carry out an objective classification of the hide defects in real time during processing of the hides.

However, the device according to this document is not capable of acquiring, either simultaneously or in alternating fashion, a pair of different images (one obtained by illuminating the grain side and the other obtained by illuminating the flesh side of the leather hide) of the same portion of the leather hide which can be compared against each other.

In fact, the device according to document DE 4216469 A1 is not capable of performing this function, as it describes a plurality of acquisition devices (16, 17) arranged aligned to one another. Whereas the light sources (24, 24') are configured to illuminate opposite portions of the leather hide (i.e. the first one from the grain side, the second one from the flesh side), the acquisition devices are configured to focus only on the upper portion of the leather hide, which can be illuminated from one side only or from both. In other words, the device according to DE 4216469 A1 does not allow the separate acquisition of an image of the same portion of a hide illuminated from the flesh side and from the grain side.

### Description of the invention

The present invention aims to eliminate or at least reduce the drawbacks and disadvantages typical of the prior art and, therefore, to propose a method and an associated apparatus for identifying possible surface defects of a leather hide which enables the acquisition of a pair of different images (one of which is obtained by illuminating the grain side and the other by illuminating the flesh side) of the same portion of the leather hide which can be compared against each other.

The method and the associated apparatus are also capable of allowing the categorisation of the leather hides on the basis of the surface defects identified and suitably classified.

One object of the present invention is to provide a method and an apparatus with a high degree of accuracy and precision in identifying possible surface defects of a leather hide. In particular, this object also comprises the possibility of configuring the apparatus capable of implementing the aforesaid method to carry out the latter continuously, without stops, unlike what needs to be done in the case of a human operator.

Another object of the present invention is to provide a method and an apparatus capable of considerably reducing the time needed to identify surface defects in order to increase productivity compared to the prior art.

A further object of the present invention is to provide a method and an apparatus capable of immediately providing information useful for categorising the hide, for example by means of the so-called "nesting" process.

The stated technical task and the specified objects are substantially achieved by a method and an associated apparatus for identifying possible surface defects of a leather hide which comprises the technical features disclosed in the independent claims. The dependent claims outline particularly advantageous embodiments of the invention.

It should be noted that this summary introduces a selection of concepts in simplified form, which will be further developed in the detailed description provided below.

Advantageously, the method according to the present invention enables the identification and classification of any surface defects that may be present to be improved in terms of accuracy, time and repeatability. In other words, compared to the prior art, this method is more consistent, i.e. it maintains the same level of quality over time, as it can be implemented constantly all day long, day after day. In addition, this method is reliable since it is capable of identifying every surface defect that exceeds a pre-set tolerance value. And, moreover, the method enables surface defects to be identified quickly, within an interval of a few seconds, and can thus improve productivity.

Furthermore, the invention relates to an apparatus for identifying surface defects of a leather hide.

In particular, the apparatus comprises a rest surface on which at least one leather hide can be positioned, a front light source configured to illuminate at least one part of a main surface of the leather hide, which is opposite relative to the rest surface, a back light source configured to illuminate at least one part of a secondary surface of the leather hide, which is facing towards the rest surface, and a scanning device having at least one viewing angle such as to frame at least one portion of the rest surface. In detail, the scanning device is configured to acquire at least a first image of the main surface of the leather hide illuminated by the front light source and a second image of the main surface of the leather hide illuminated by the back light source. In particular, the scanning device and the rest surface are disposed in mutual relative motion along a longitudinal direction.

The apparatus further comprises a control unit which is connected to at least one scanning device and is programmed to identify the presence of possible surface defects on each acquired image of the leather hide. More precisely, the control unit is configured to carry out the previously described method for identifying surface defects.

Advantageously, the apparatus enables a leather hide to be scanned with a high level of definition (for example, it can discriminate details of 1 mm²). In particular, said apparatus is capable of acquiring an overall surface image of the leather hide consisting of two different types of images: a first substantially classic image (albeit with a higher level of detail and thus qualitatively better, being independent of the operator), i.e. one obtainable through normal direct vision, and a second image similar to an X-ray, in which many classes of defects otherwise invisible in the first image are rendered visible.

### Brief description of the drawings

Additional characteristics and advantages of the present invention will emerge more clearly from the approximate, and therefore not limiting, description of a preferred but not exclusive embodiment of a method and an associated apparatus for identifying possible surface defects of a leather hide, as illustrated in the accompanying drawings, in which:
- figure 1 illustrates a block diagram representative of a method for identifying possible surface defects of a leather hide;
- figure 2 illustrates a block diagram representative of the training of the surface pattern analysis software based on machine learning algorithms;
- figure 3 illustrates a block diagram representative of a process for categorising leather hides comprising the method for identifying possible surface defects;
- figure 4 illustrates a list of surface defects with which an identification code and a quality code are associated;
- figures 5a-5c illustrate the various parts which define the vector file generated in step 131 of the process 1000;
- figure 6 illustrates a table used for the categorisation of a leather hide by a nesting program;
- figure 7a illustrates, according to a side view, a possible embodiment of the apparatus for identifying possible surface defects of a leather hide;
- figure 7b illustrates an enlarged view of a portion of the apparatus illustrated in figure 7a;
- figure 8 illustrates, according to a top view, the apparatus shown in figure 7a.
With reference to the drawings, they serve solely to illustrate embodiments of the invention.

### Description of one embodiment

The present invention relates to an apparatus for the surface scanning of leather hides, as well as a method for identifying surface defects of a leather hide which is implemented by means of said apparatus.

Possible modifications or variants which, in the light of the description, should be evident to the person skilled in the art must be considered as falling within the scope of protection established by the present invention, according to considerations of technical equivalence.

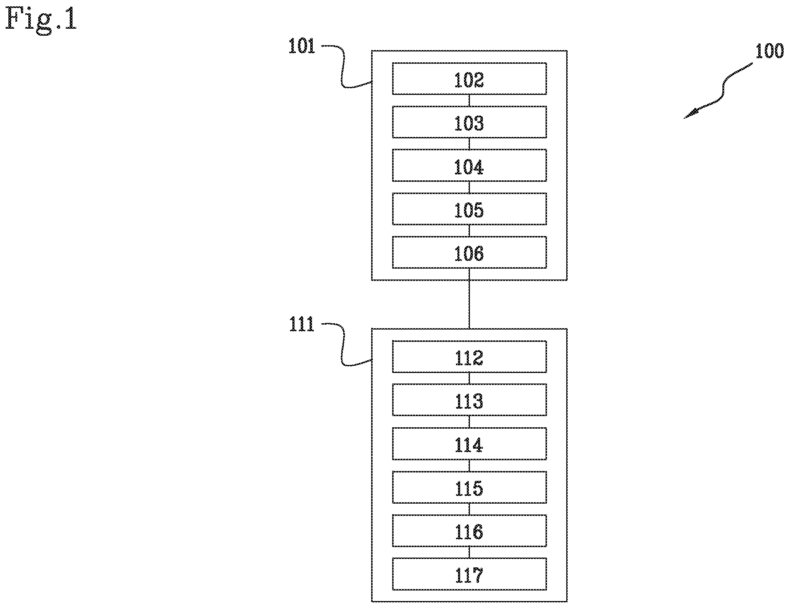

Figure 1 illustrates a block diagram representative of a method 100 for identifying possible surface defects of a leather hide placed on a rest surface 2.

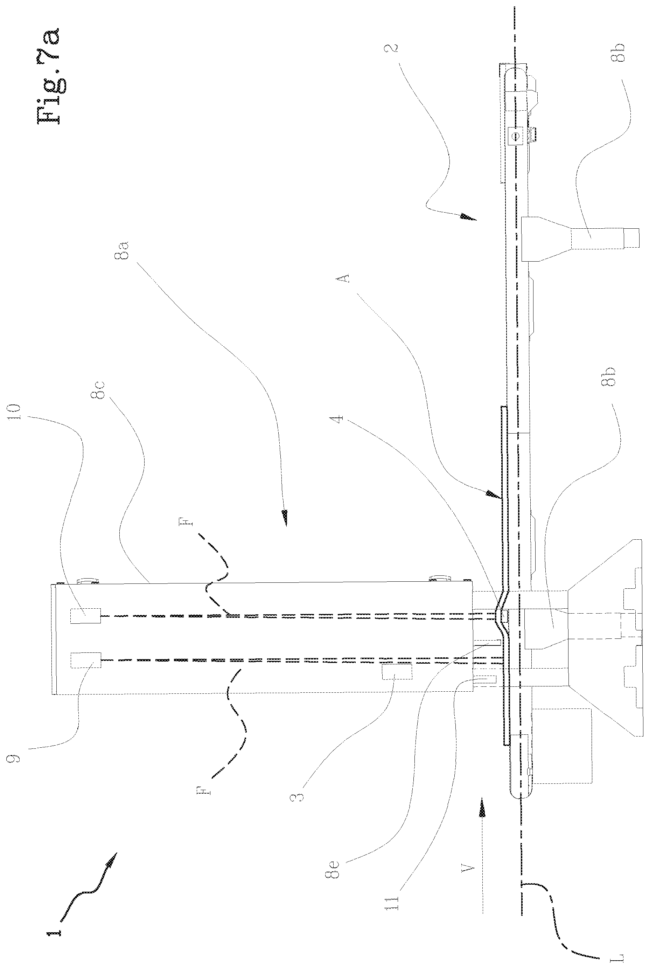

According to one embodiment, the method 100 is implemented by means of an apparatus 1 for identifying possible surface defects of a leather hide illustrated in figures 7a-8 and subsequently described in greater detail.

Schematically, the method 100 comprises at least a first acquisition step 101 for acquiring at least one surface image of a leather hide and a second step 111 for analysing the aforesaid acquired surface image in order to identify possible surface defects.

In particular, the first acquisition step 101 comprises, in turn, the following steps:
- illuminating, with at least one front light source 3 in front of the leather hide, at least one part of a main surface A of the same leather hide, which is opposite the rest surface 2 (step 102);
- illuminating, with at least one back light source 4 at the back of the leather hide, at least one part of a secondary surface of the same leather hide, which is facing towards the rest surface 2 (step 103);
- acquiring, with a scanning device 5 comprising a first series of line scan cameras 9, at least a first image of the main surface A of the leather hide illuminated by the front light source 3 (step 104). In detail, the first image is representative of a first series of surface characteristics of the leather hide. In greater detail, said first series of line scan cameras 9 of the scanning device 5 has at least a viewing angle F, such as to frame a portion of predetermined width of the rest surface 2 in order to acquire at least one image of the main surface A of the leather hide;
- acquiring, with the scanning device 5 comprising a second series of line scan cameras 10, which are offset relative to the first series of line scan cameras 9, at least a second image of the main surface A of the leather hide illuminated by the back light source 4 (step 105). In detail, said second series of line scan cameras 10 of the scanning device 5 has a viewing angle F such as to frame a portion of predetermined width of the rest surface 2 in order to acquire at least one image of the main surface A of the leather hide, the second image being representative of a second series of surface characteristics of the leather hide.

Step 111 for analysing the acquired surface images comprises at least a step of comparing, with a control unit (not illustrated), the first image and the second image with at least one reference image or model contained in a storage unit in order to identify possible surface defects of the leather hide. In detail, the reference image or model are representative of a series of reference surface characteristics of a leather hide with and/or without surface defects. In particular, the control unit is connected to the storage unit and to the scanning device 5.

The control unit, being advantageously connected to the scanning device 5, is configured to perform a comparison between each pair of images acquired for each leather hide and one or more reference images present in a storage unit, likewise connected to the control unit.

In this manner, the method 100 is advantageously capable of identifying the presence, if any, and positioning of the known surface defects on each scanned leather hide.

Generally, as may be deduced from the part that follows of the present description, each acquired image of the leather hide contains a large amount of information to be processed, for example in the order of about 70 megapixels, which is advantageously analysed in a short time by the control unit and with a high degree of accuracy so as to detect at least the majority of the possible surface defects present, preferably all of them, irrespective of their nature, shape and extent. In contrast, as noted previously, the prior art shows multiple limits deriving from the execution of a manual procedure, including: the need to use specialised operators, lengthy working times for each leather hide and subjective results of the evaluations depending on the specialised operator who has carried them out.

According to an important feature of the invention, the acquisition step 101 of the method 100 envisages that at least one pair of images is acquired for every leather hide positioned on the rest surface 2, each having a predetermined width defined by the width of the acquisition angle F of the respective cameras 9, 10 of the scanning device 5. More precisely, the first image is obtained by scanning a portion having a predetermined width, defined by the width of the acquisition angle F, of the main surface A of the leather hide, i.e. of the so-called "grain" side, which is directly illuminated by a front light source 3. The second image, by contrast, is obtained by scanning a portion having a predetermined width, defined by the width of the acquisition angle F, of the main surface A of the leather hide when the latter is backlit by means of the back light source 4, i.e. it is illuminated with a light source facing directly towards verso the so-called "flesh" side of the leather hide.

Advantageously, the acquisition of the first image and second image can take place simultaneously, since different parts of the main surface A of the leather hide are illuminated and scanned simultaneously, or in an alternating fashion, as it could be possible to illuminate the leather hide with one light source at a time.

Even more advantageously, providing both a frontal illumination and a backlighting of the leather hide makes it possible to obtain two distinct images in which different surface defects are visible, if present. Some classes of surface defects, in fact, are considerably harder to identify if only the first image obtained by direct illumination of the "grain" side of the leather hide is available. For example, surface defects such as deep prick marks, open warble holes or blind warble holes with a diameter greater than about half a millimetre are more easily identifiable with the acquisition of a backlit image of the leather hide.

According to one aspect of the invention, the first acquisition step 101 of the method 100 also comprises the step 106 of moving the scanning device 5 and/or the rest surface 2 along a longitudinal direction L. In particular, the scanning device 5 is configured to frame a pair of portions of predetermined widths of the main surface A of the leather hide based on the reciprocal movement between the rest surface 2 and the respective series of line scan cameras 9 10 of the scanning device 5.

In other words, the reciprocal motion along the longitudinal direction L between the rest surface 2 and the scanning device 5 enables the scanning device 5 itself to totally acquire the main surface A of the leather hide with a series of successive images also in the event that the acquisition angle F is capable of framing only a part of the main surface A.

Preferably, step 106 envisages maintaining the scanning device 5 in a static position, while the rest surface 2 is moved along the longitudinal direction L according to an advancement direction V.

Even more preferably, as better described below, the rest surface 2 comprises a motorised conveyor belt 7 capable of advancing, and thus conveying, a leather hide along the advancement direction V towards the viewing angle F of the scanning device 5.

According to a preferred aspect of the invention, step 106 comprises a sub-step of synchronising the movement between the rest surface 2 and the scanning device 5 based on a value of frequency of acquisition of the first image and/or second image by the first series of line scan cameras 9 and second series of line scan cameras 10 of the scanning device 5.

In this manner, the scanning device 5 is capable of acquiring, without distortions, at least a first and a second image of the leather hide with a definition sufficiently high to enable the control unit to perform a comparison with the reference images in the storage unit.

In fact, if the movement of the leather hide relative to the scanning device 5 takes place at too high a speed compared to the frequency of acquisition of the images (i.e. compared to the speed at which the scanning device 5 is capable of acquiring the image), the pixels that form the acquired image could be deformed compared to their normal optimal square shape.

According to the invention, step 104 of acquiring a first image of the leather hide with the scanning device 5 comprises the further sub-steps of:
- acquiring, by means of a first series of line scan cameras 9, a first plurality of two-dimensional images of the leather hide illuminated by the front light source 3 based on the value of the frequency of acquisition of said line scan cameras 9 of the scanning device 5 and the reciprocal movement between the rest surface 2 and the scanning device 5;
- sending the first plurality of two-dimensional images to the control unit so as to join them together to obtain a first image of the leather hide consisting of the union of a series of images acquired by the line scan cameras 9, having a predetermined width defined by the width of the acquisition angle F and stitched to one another to form a continuous strip.

According to the invention, step 105 of acquiring a second image of the leather hide with the scanning device 5 comprises the further steps of:
- acquiring, by means of a second series of line scan cameras 10, a second plurality of two-dimensional images of the leather hide illuminated by the back light source 4 based on the value of the frequency of acquisition of said line scan cameras 10 of the scanning device 5 and the reciprocal movement between the rest surface 2 and the scanning device 5;
- sending the second plurality of two-dimensional images to the control unit so as to join them together to obtain a second image of the leather hide consisting of the union of a series of images acquired by the line scan cameras 10, having a predetermined width defined by the width of the acquisition angle F and stitched to one another so as to form a continuous strip.

The main advantage deriving from the use of line scan cameras, i.e. cameras having a CCD sensor made up of a single line of photosensitive elements, compared to the more common matrix cameras, is the guarantee of acquiring high-resolution images in order to ensure high quality. Given the nature of the camera sensor, it is capable of scanning a sole linear portion of the leather hide at a time, i.e. each of the previously mentioned two-dimensional images. Therefore, it becomes necessary to induce a reciprocal movement between the camera and the leather hide in order to vary the framed part of the main surface A of the leather hide over time. Subsequently, the union of each two-dimensional image generated by the first series of line scan cameras 9 and the second series of line scan cameras 10 enables the reconstruction of an overall image of the leather hide.

Preferably, the movement of the leather hide takes place by virtue of the movement of the rest surface 2, for example a motorised conveyor belt 7, relative to the static position of the scanning device 5 comprising the first series of line scan cameras 9 and the second series of line scan cameras 10 in order to maintain the position of the image acquisition device and of the light source associated therewith fixed and stable.

According to one aspect of the invention, step 104 of acquiring the first image and step 105 of acquiring the second image are carried out simultaneously. In particular, in fact, the scanning device 5 comprises a first series of line scan cameras 9 for acquiring the first image and a second series of line scan cameras 10 for acquiring the second image.

In this manner, during the reciprocal motion between the rest surface 2 (preferably mobile) and the scanning device 5 (preferably static), each of the two series of line scan cameras 9, 10 is configured to frame a respective part having a predetermined width, defined by the width of the acquisition angle F, of the main surface A of the leather hide illuminated by a respective light source 3, 4.

Advantageously, in this manner it is possible to simultaneously acquire the first and second images of the main surface A of the leather hide.

According to one aspect of the invention, step 102 of illuminating with the front light source 3, step 103 of illuminating with the back light source 4, step 104 of acquiring the first image and step 105 of acquiring the second image are carried out by the control unit on the basis of a position signal received from at least one position sensor 11. More precisely, each position sensor 11 is configured to detect the presence of the leather hide within the viewing angle F of the scanning device 5.

In particular, at least one position sensor 11 is provided at the scanning device 5 and is positioned relative to the rest surface 2 in such a way as to detect when the leather hide is in proximity to the portion of predetermined width defined by the acquisition angle F of the same scanning device 5.

In other words, when at least one position sensor 11 detects the presence of the leather hide in proximity to the portion of predetermined width defined by the acquisition angle F of the scanning device 5, the control unit, which receives a detection signal from the position sensor 11, is configured, on the basis of that signal, to activate the illumination of the leather hide and the acquisition by the scanning device 5 in order to obtain the first and second images of the main surface A, which can be advantageously joined in a single overall image analysable by the surface pattern analysis software.

According to one aspect of the invention, step 111 of analysing the acquired surface images by means of the comparison performed by the control unit comprises the following additional steps:
- receiving at least the first image and the second image from the scanning device 5 (step 112);
- running a surface pattern analysis software application programmed to compare the first image and the second image with each reference image or model contained in the storage unit (step 113);
- detecting possible surface defects present on the leather hide from the first series of surface characteristics of the first image and/or from the second series of surface characteristics of the second image (step 114);
- sending an output signal representative at least of the presence and/or absence of the possible surface defects identified to an external display device (step 115).

Advantageously, step 111 for analysing the surface images of the method 100 is based on the use of automatic objective techniques for the detection of surface defects, so as to considerably increase the quality standards of the analysis. Consequently, the method 100 makes it possible to obtain a reduction in production costs by virtue of a correctly defined use of the leather hides and a drastic reduction of rejected finished product, since in this manner use is made only of the portions free of surface defects or in which the surface defects identified are tolerable.

A first aspect of the invention envisages that the images contained in the storage unit represent images of leather hides or portions of leather hides in which one or more surface defects are present so that the control unit is capable of defining the type of surface defect through a simple comparison of images.

A second and preferred aspect of the invention envisages that each image contained in the storage unit is used to define, preferably by means of artificial intelligence algorithms (as explained below), a reference model for each possible type of surface defect, so that the control unit is capable of defining the type of surface defect by searching for the correct reference model with which to associate the surface defect in the acquired image of the leather hide.

According to another aspect of the invention, the method 100 comprises a step 116 of storing at least the first image and/or the second image in the storage unit.

In this manner, for each scanned leather hide, the respective first image and second image are stored in the storage unit to expand the database used by the surface pattern analysis software to perform a comparison between the acquired images and the reference images.

In other words, the images acquired and compared can be stored in order to be used as standards of reference.

Preferably, the control unit is configured to store the first and second images of the leather hide after having analysed them, i.e. after step 114 of detecting possible surface defects.

Even more preferably, as better explained below, the control unit, following step 114 of detecting surface defects, is configured to generate a file containing both the images acquired for the respective leather hide and the metadata referring to those images, which in turn contain the characteristic data of the surface defects identified, for example their location, shape, size, classification ...

According to one embodiment of the invention, the control unit is configured to run a surface pattern analysis software application based on machine learning algorithms, for example ViDi^{®} software by the Cognex^{™} company. The software is configured to train a neural network of the VODNN type, i.e. a "Vision Optimized Deep Neural Network", capable of locating and identifying characteristics within images, locating and reading characters within images, identifying, locating and characterising defects in the images and classifying the same images.

Normally, in fact, a leather hide, as a natural product, exhibits high structural variability and, consequently, its surface defects likewise exhibit irregularities in their physical characteristics, in terms of both appearance and size, such as to make it very difficult to construct exact models for their recognition by human operators or by mean of rules-based image computing and analysis programs.

Advantageously, unlike image computing and analysis programs based on rules and the use of filters, surface pattern analysis software based on machine learning algorithms provides for the creation of neural networks capable of learning from a series of examples provided by the user, for example from a series of training data and images representative both of surface defects and of the absence thereof. Even more advantageously, the neural networks used by the surface pattern analysis software are capable of distinguishing the characteristics of an image while simultaneously tolerating surface variations within certain tolerance limits pre-set by the operator.

In greater detail, the surface pattern analysis software used comprises four categories of parameters that are useful for performing a precise calibration of performance and of the way in which the images are processed.

The sampling category comprises:
- the characteristic size of the surface defects, i.e. the diameter, in pixels, of the characteristics to be looked for;

- their characteristic colour, which, in the case of the present invention is sampled both for a colour image, the first image, and for a greyscale image, the second image;
- the type of edge, specifying how the outer pixels must be sampled; and,
- masking for specifying whether a mask or graphic filter must be applied for certain functions. For example, the area outside the main surface A of the leather hide can be eliminated by means of a mask to optimise processing speed.

The training category comprises:
- the set of reference images to be used as a standard of comparison and the percentage of images to be used, after a random selection, for every training process;
- the count of "epochs", i.e. the number of optimisation iterations carried out during training; and,
- the ability to consider the different degrees of visual complexity.

The perturbation category comprises parameters such as rotation, scale, appearance, luminance and contrast, which are useful for artificially generating training images.

Finally, the processing category comprises:
- the density of the sampling points with respect to the instrument setting of the size of the characteristics;
- the minimum score thresholds that can be used in order to be able to classify an identified surface defect;

- the iterations for searching for the most evident anomalies; and,
- the region filter to be used as a criterion for correctly marking the regions found (e.g. score, area, perimeter, compactness).

In addition, the control unit is preferably configured to apply a first graphic filter, whose purpose is to eliminate the background, and a second graphic filter, such as to eliminate the folds in the leather hide, i.e. the shadow zones that must not be considered as surface defects. Even more preferably, the folds in the leather hide are recognisable by means of suitable training with examples implemented by the surface pattern analysis software.

As mentioned earlier, according to one aspect of the invention, the surface pattern analysis software based on machine learning algorithms envisages a step of training the neural network.

Advantageously, the surface pattern analysis software comprises both a training step in a supervised mode, and a training step in an unsupervised mode.

In the first supervised mode, for each class of defects a respective identifying image is acquired and stored in the storage unit. The surface pattern analysis software is made to analyse the reference images in which the surface defects are highlighted and labelled and, subsequently, also the images of leather hides or parts thereof free of surface defects. In this manner, the neural network is capable of creating a first model of surface defect identification and classification which will have to be validated with further acquired images of leather hides in which the surface defects are not highlighted or labelled.

In the second unsupervised training mode, the surface pattern analysis software is configured to learn the appearance of parts in which surface defects are present in comparison with preloaded parts of the leather hide free of surface defects. Preferably, the second training mode is used downstream of the first training mode.

Even more preferably, once the surface pattern analysis software has been trained and, therefore, the model used by the neural network to identify and classify surface defects has been validated, it will assign a label to each image analysed to indicate the percentage of precision in the classification of surface defects.

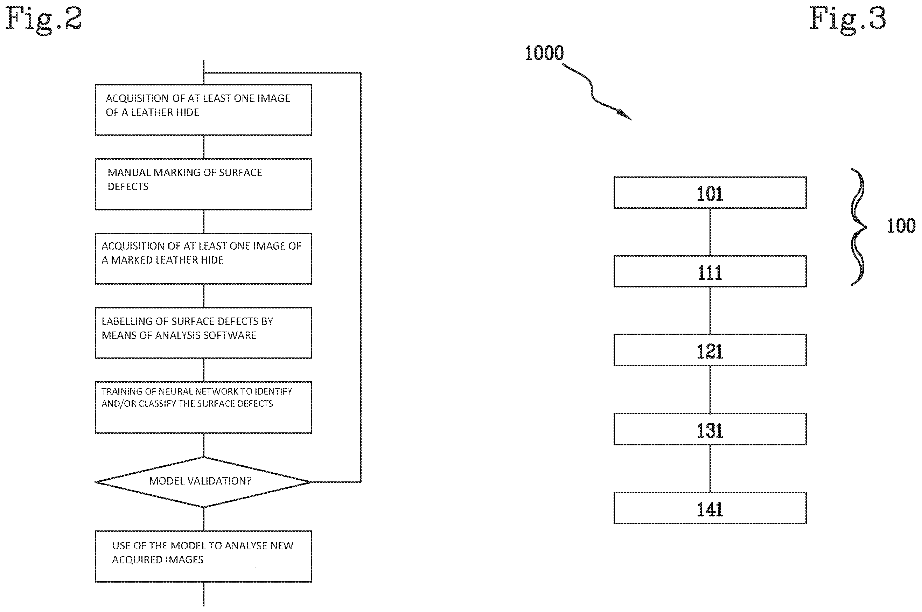

Figure 2 illustrates a block diagram representative of the training of the surface pattern analysis software, which, during training in the supervised mode, comprises:
- acquiring at least one image of the main surface of a leather hide;
- manually marking the leather hide to identify the surface defects;
- acquiring at least one image of the main surface of the manually marked leather hide. The marked leather hides have been scanned so as to be used as reference images by the surface pattern analysis software once the reference images of each class of surface defects have been provided;
- labelling the surface defects by means of an external device, such as a processor, personal computer, tablet;
- analysing the first acquired image with the surface pattern analysis software in order to verify whether the identification and classification model has been validated.

If it has, the model can be used to analyse new images of new leather hides. At this point it will be possible to implement unsupervised training to recalibrate the precision of the model in identifying and discriminating the surface defects.

If it has not, the training step will be reiterated until the model is validated.

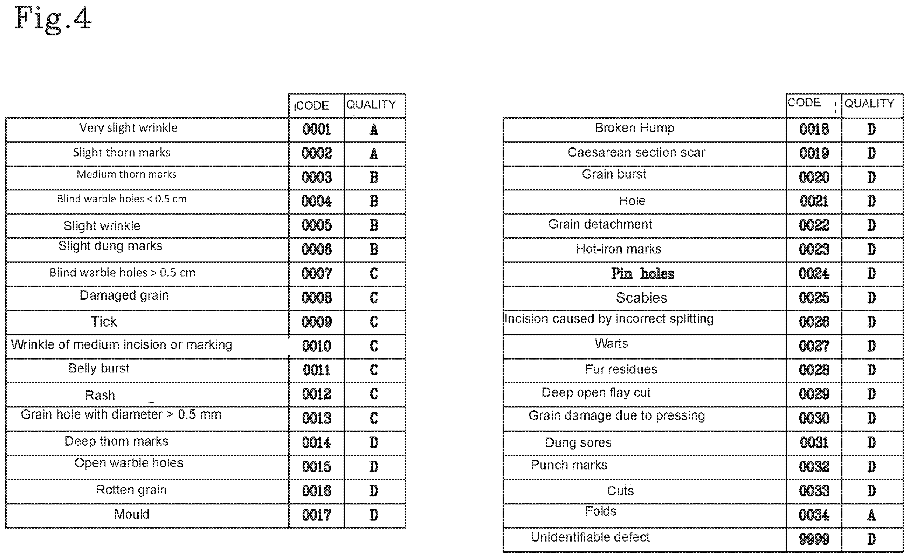

Advantageously, both for the training of the neural network and for the identification and classification of surface defects, a list was drawn up (which may be seen in figure 4) of all known surface defects, such as wrinkles, prick marks, warble holes, damaged grain, mould, holes, etc.... Each defect was then assigned a recognition code which can be used by the surface pattern analysis software and a quality code, i.e. a further code identifying the type of surface defect that can be used for the step of categorising the leather hide (explained further below in the description).

Even more advantageously, several dozen leather hides were scanned to provide a sufficient number of reference images for each class of surface defects. Preferably, each leather hide was also carefully analysed by a specialised operator, who marked the surface defects present with a felt-tip marker, with an indication of the relevant code.

According to another aspect of the invention illustrated in figure 1, the method 100 comprises a step 117 of joining the first image and the second image in an overall image representative both of the first series of surface characteristics and of the second series of surface characteristics of the leather hide.

Advantageously, the overall image is thus capable of highlighting both the classes of surface defects identifiable with front illumination and the classes of surface defects identifiable with backlighting, which provides an image similar to an X-ray and, therefore, generally not visible to the human eye.

According to one aspect of the invention, the control unit comprises a first analysis module configured to analyse the first image of the leather hide illuminated from the front and a second analysis module configured to analyse the second backlit image of the leather hide. In this manner, following the analysis, the control unit is advantageously configured to generate an overall image of the leather hide in which both the defects visible in the first image and those visible in the second image are shown.

Figure 3 illustrates a process 1000 for categorising a leather hide, which comprises the previously described method 100 for identifying surface defects.

This process, in addition to the already described step 101 for acquiring at least one surface image of a leather hide and step 111 for analysing the aforesaid surface image, comprises a step 121 for classifying the detected surface defects, a step 131 for generating a vector file representative of the physical characteristics and metadata of the scanned leather hide and the identified and classified surface defects thereof, and, finally, a step 141 for categorising the scanned leather hide on the basis of the data contained in the vector file generated in step 131.

Step 121 for classifying the identified surface defects is preferably carried out by means of the already mentioned surface pattern analysis software.

Advantageously, in the case of use of software based on machine learning algorithms, the software itself is trained both to identify the surface defects, and to classify them, as it is configured to discriminate both the presence of a defect and the characteristics thereof which makes it such and, therefore, it is possible to differentiate between one class and another of surface defects.

Step 131 for generating a vector file is an immediate consequence of the identification step 111 and/or the classification step 121.

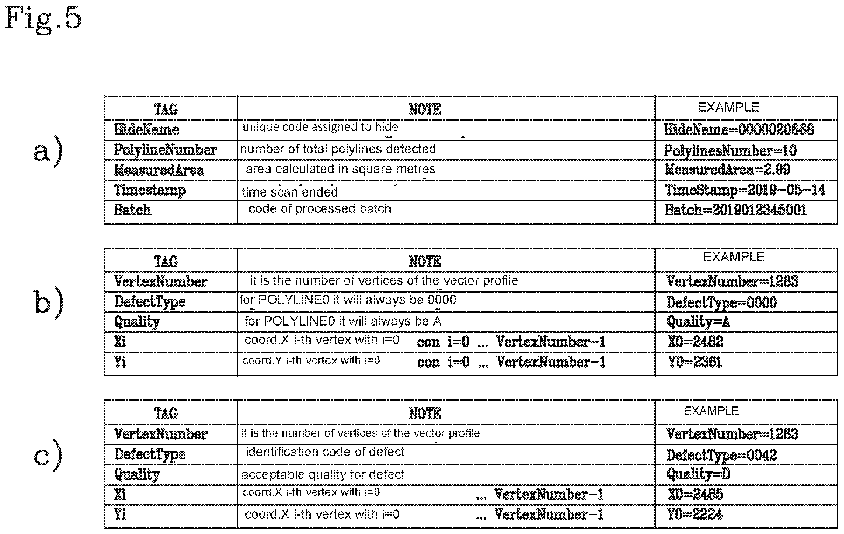

The vector file generated in said step 131, preferably an INI file, represents sets of coordinates of the scanned main surface A of the leather hide and the detected surface defects. An example of said vector file is shown in figures 5a-5c.

More precisely, the vector file comprises a fixed section, called [HIDEINFO], as can be seen in figure 5a, which contains information about the scanned leather hide, for example the batch it belongs to, the date of the scan ..., and a plurality of variable sections, called [POLYLINEi], as can be seen in figures 5b, 5c, for each detected geometric profile, i.e. for each detected surface defect, in which the coordinates of the detected profile thereof are memorised. The character "i" present in [POLYLINEi] represents any variable section recorded and varies between "0" and "n", where "n" corresponds to the maximum number of surface defects detected.

Even more precisely, the first variable section called [POLYLINE0], as can be seen in figure 5b, comprises information about the part of main surface A of the leather hide free of surface defects, whilst the subsequent variable sections [POLYLINEi], as can be seen in figure 5c, with "i" varying between "1" and "n", comprise information about the profiles of the surface defects detected.

Step 141 for categorising the scanned leather hide is useful for maximising the usable surface of the leather hide and, therefore, minimising the waste of valuable raw materials.

Manually, or in an automatic mode, a nesting process comprises maximising the number of rectangular templates or outlines that can be contained within the main surface A of the leather hide without overlapping them on the detected surface defects, or at least a part of them. As mentioned previously, in fact, some parts of the leather hide in which some classes of defects are present can nonetheless be used to manufacture a secondary product.

Generally, therefore, the categorisation of a leather hide is based on three factors:
- the surface extent of the templates used;
- the amount of templates that can be positioned on the leather hide;
- the total surface area of the leather hide.

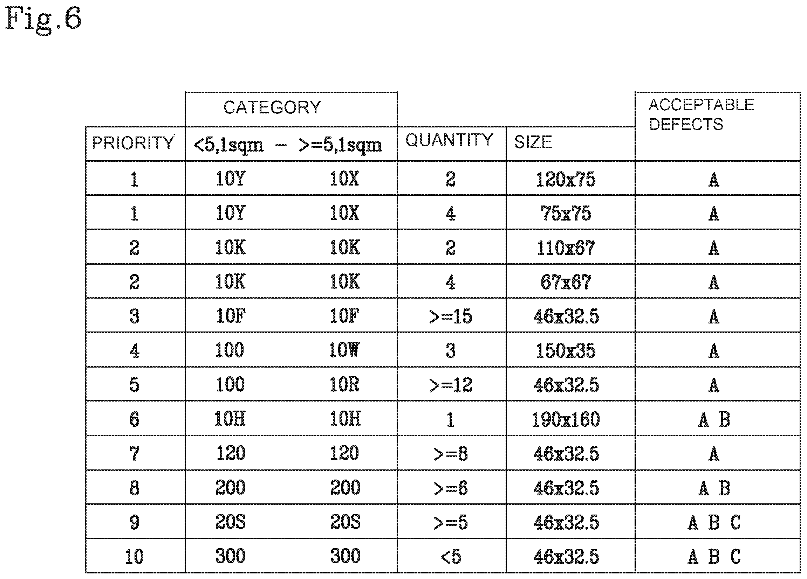

The table illustrated in figure 6 shows the criteria used by the nesting process to determine the category of the leather hide analysed. This table is ordered according to a level of priority so that the nesting process will apply a determination algorithm configured to try to associate the highest quality category with the leather hide. If one of the conditions is not met, the iteration algorithm will pass on to the next highest quality category iteratively until reaching a valid categorisation value. In other words, the leather hide is categorised based on the best result obtainable and obtained.

For example, a hide with an upper surface of 5.1 m² is classified as category "X" if two templates having a width and length equal to 120 cm and 75 cm or four templates having a width and length equal to 75 cm can be overlaid on the scanned main surface A thereof.

As may be seen from the last column on the right of the table in figure 6, some surface defects are tolerated in determining the leather hide categories. More precisely, the surface defects associated with a precise quality code, which is shown in the table in figure 6, are tolerated.

For example, in reference to the table in figure 6, the surface defects whose quality code is "C" are allowed only for the categories "20S" and "300", in which the leather hides are capable of containing less than five templates having a size, in terms of width and length, of 46 cm x 32.5 cm.

The invention further relates to an apparatus 1 for identifying surface defects of a leather hide.

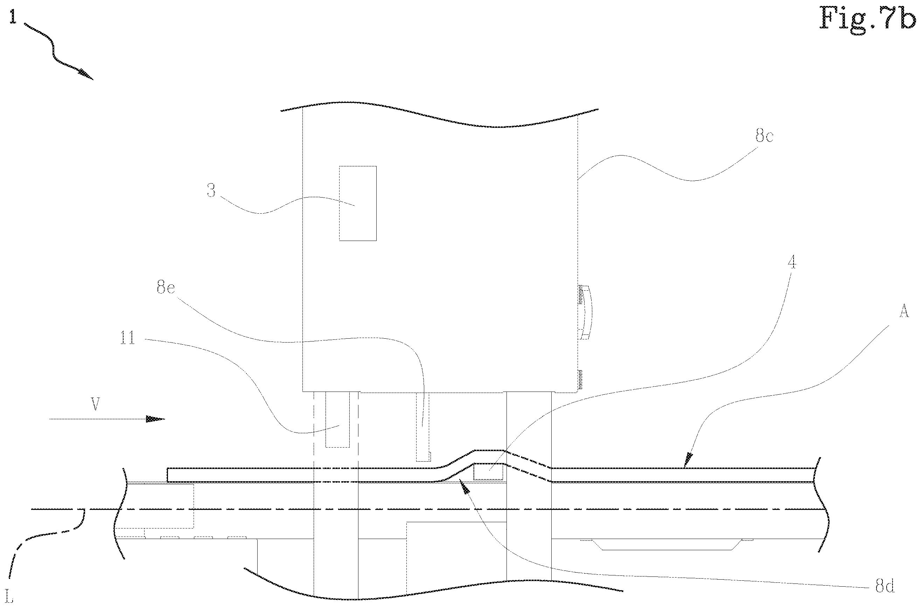

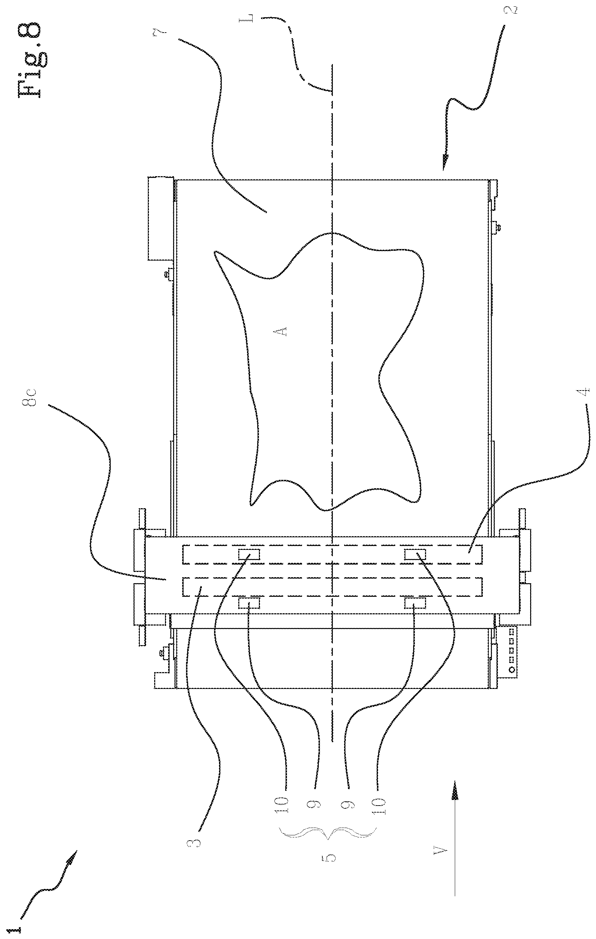

Figures 7a, 7b, 8 illustrate the apparatus 1, which comprises a rest surface 2 on which at least one leather hide can be positioned, a front light source 3, a back light source 4, a scanning device 5 consisting of a first series of line scan cameras 9 and a second series of line scan cameras 10, the second series being arranged longitudinally offset from the first series, and a control unit (not illustrated) connected at least to the scanning device 5 and programmed to identify the presence of possible surface defects in each acquired image. In particular, the control unit is configured to implement the previously described method 100 for identifying surface defects.

The line scan cameras 9 and 10 of the scanning device 5 possess a viewing angle F such as to frame two distinct portions of the rest surface 2 having predetermined widths.

As described previously, the rest surface 2 and/or the scanning device 5 are moved along a longitudinal direction L.

Furthermore, according to one aspect of the invention, the rest surface 2 comprises a motorised conveyor belt 7 which extends along the longitudinal direction L and is configured to convey the leather hide forward along the same longitudinal direction L, according to an advancement direction V.

Preferably, the rest surface 2 has an extent such as to allow the positioning of at least one leather hide having a length of about 4 metres and a width of about 3 metres.

Even more preferably, the rest surface 2 has a dark colour, generally black, in order to provide a background that contrasts with the colour of the leather hide to be scanned so as to be able to acquire an image of the latter with the contours better delineated.

Advantageously, therefore, the dark background makes it possible to have the leather hide stand out in the image, from a chromatic viewpoint, in order to correctly apply the filter for eliminating the background and analyse solely the parts of the image referring to the leather hide.

According to one aspect of the invention, the apparatus 1 comprises a support frame 8a to which at least the rest surface 2 and the scanning device 5 are connected. Preferably, the support frame 8a comprises a plurality of support legs 8b and a box-like body 8c positioned above the rest surface 2.

In particular, the motorised conveyor belt 7 is connected to the support legs 8b in such a way as to be kept raised from the floor by about 90 cm and the upper portion thereof, on which the leather hide is placed, is made to advance by movement means, for example motorised rollers, along the advancement direction V.

The box-like body 8c, by contrast, extends vertically relative to the rest surface 2 to enable the support of the first series of line scan cameras 9 and second series of line scan cameras 10 of the scanning device 5, whose viewing angle F is turned towards the motorised conveyor belt 7. Preferably, the front light source 3 and the position sensors 11 are also connected to the box-like body 8c. In this manner, the front light source 3 is capable of illuminating the main surface A of the leather hide, whilst the position sensors 11 are capable of detecting the passage of the leather hide.

According to one aspect of the invention, the back light source 4 is positioned above the motorised conveyor belt 7, almost in contact with the latter. Present adjacent to it there is an inclined ramp 8d connected to the support frame 8a and configured to lift the leather hide so that the latter passes over the back light source 4 in order to be backlit.

Alternatively, according to a different aspect of the invention, not illustrated, the back light source 4 is interposable between two motorised conveyor belts 7 situated side by side, both extending along the longitudinal direction L, and configured to convey the leather hide forward correctly along the same advancement direction V.

Advantageously, the box-like body 8c is positioned above the motorised conveyor belt 7 in a static position so that the line scan cameras 9, 10 of the scanning device 5 turn their viewing angle F in order to frame two distinct portions of the leather hide having a predetermined width. Thanks to the advancement of the motorised conveyor belt 7 and, therefore, of the leather hide, the line scan cameras 9, 10 of the scanning device 5 are capable of intercepting the entire main surface A of the leather hide illuminated by the front light source 3 and, therefore, of acquiring an entire image of the same leather hide.

Preferably, the motorised conveyor belt 7 comprises a speed variator configured to vary the advancement speed between a minimum of 15 metres per minute to a maximum of 50 metres per minute. Preferably, the motorised conveyor belt is configured to advance with a speed of 25 metres per minute, in order to ensure the scanning of a leather hide along about 3 metres in about 7 seconds.

According to one aspect of the invention, the apparatus 1 comprises a synchronisation device (not illustrated) associated with the rest surface 2 and with the scanning device 5. In particular, the synchronisation device is configured to synchronise their reciprocal motion based on a value of frequency of acquisition of the images of the scanning device 5.

Preferably, the apparatus 1 comprises an encoder configured to synchronise the advancement speed of the motorised conveyor belt 7 with the acquisition speed of the scanning device 5, i.e. the scanning frequency value thereof, in order to acquire an undistorted image in which the pixels are square in shape and not elongate.

In other words, the scanning device 5 is preferably maintained in a stable, static position, whilst the rest surface (a motorised conveyor belt 7) is moved along the longitudinal direction L according to an advancement direction V.

Figures 7a and 7b illustrate a preferred positioning of the light sources of the apparatus 1.

Preferably, the front light source 3 is configured to illuminate at least a part of a main surface A of the leather hide, the so-called "grain" side opposite the rest surface 2. The back light source 4, by contrast, is configured to illuminate at least one part of a secondary surface of the leather hide, the so-called "flesh" side facing the rest surface 2.

According to one aspect of the invention, the scanning device 5 acquires a colour image as the first image of the leather hide, since the front light source 3 is preferably an LED lamp capable of illuminating with diffused white light at a temperature of 5000 K.

Alternatively, according to an unillustrated aspect of the invention, both the front light source 3 and the back light source 4, preferably an LED, can be configured to emit a collimated, direct light beam on the leather hide. In this manner, both light sources can be turned towards the leather hide so that the light beam is inclined by about 45° relative to the rest surface 2 on which the leather hide is placed. Advantageously, the provision of oblique rather than perpendicular lighting enables images with a higher quality to be acquired.

In particular, according to the latter alternative embodiment, a possible arrangement of the light sources envisages that the front light source 3 comprises a pair of LED sources emitting uniform white light, positioned opposite each other and inclined by an angle of 45° relative to the portion of the main surface A of the leather hide to be illuminated frontally. The angle makes it possible to have an oblique light effect capable of improving the quality of the first acquired image and simultaneously to reduce the effect of "flattening" of surface irregularities which can derive from a substantially perpendicular illumination with an LED diffused light source. Advantageously, the arrangement of two opposite LED sources makes it possible to avoid possible shadow effects deriving from the use of a single angled source. In this configuration, the positioning of the back light source 4 remains unchanged and, therefore, entails positioning the LED point source facing towards the secondary surface of the leather hide in order determine a backlighting on the so-called "flesh side". Durante the backlighting, the leather hide is able to advance, passing over the back light source 4 so that the scanning device 5 acquires the second image.

According to one aspect of the invention, the scanning device 5 acquires a greyscale image as the second image of the leather hide, since the back light source 4 for backlighting is preferably an LED lamp capable of illuminating with uniform white light at 6000 K. Advantageously, the use of the backlighting technique to acquire the second image of the leather hide makes it possible to obtain an image similar to an X-ray, in which some classes of surface defects harder to identify in the first image are more visible.

As described above, the scanning device 5 comprises at least a first series of line scan cameras 9, configured to frame a first portion of predetermined width (substantially, as better explained below, a linear portion) of the main surface A of the leather hide illuminated by the front light source 3. In particular, the first series of line scan cameras 9 is configured to acquire a first plurality of two-dimensional images relating, respectively, to the first portion of the leather hide, according to the reciprocal motion between the rest surface 2 and the scanning device 5.

Furthermore, the scanning device 5 comprises a second series of line scan cameras 10, configured to frame a second portion of predetermined width of the main surface A of the leather hide. Therefore, the first portion is illuminated by the front light source 3 and the second portion is illuminated by the back light source 4. In particular, the second series of line scan cameras 10 is configured to acquire a second plurality of two-dimensional images relating to the second part of the leather hide, according to the reciprocal motion between the rest surface 2 and the scanning device 5.

In other words, the scanning device 5 comprises two series of line scan cameras 9, 10 configured to focus on different portions of the leather hide, which are illuminated by different light sources. In this manner, the scanning device 5 according to the present embodiment is advantageously capable of simultaneously acquiring both the first image and the second image of the leather hide.

Preferably, irrespective of the positioning of the light sources, the apparatus 1 comprises a separating partition 8e between the light sources 3, 4 to prevent the illumination generated by one light source from contaminating the portion of the hide illuminated by the other light source and, therefore, altering the scanning of the image by the respective image acquisition device.

Preferably, the first line scan cameras 9 are set side by side and lie in a same first vertical plane, which is orthogonal relative to the longitudinal direction L. The second line scan cameras 10 are likewise set side by side and lie in a same second vertical plane, which is orthogonal relative to the longitudinal direction L, but said second plane is parallel and distanced from the first plane by a predefined distance, preferably about 40 centimetres.

Advantageously, the second series of line scan cameras 10 and the back light source 4 lie in the same plane on opposite sides of the leather hide, so that the respective viewing angle F frames the backlit portion of the main surface A of the leather hide.

Preferably, the first series of cameras 9 and the front light source 3 are arranged unaligned and lie in two respective parallel planes distanced from each other, for example by about 15 centimetres, so that the front light source 3 is positioned outside the viewing angle of the first pair of cameras 9 so as not to cause an impediment to the acquisition of the first image of the main surface A of the leather hide.

Advantageously, the front light source 3 is an LED diffused light source capable of illuminating the portion of the main surface A of the leather hide framed by the first series of line scan cameras 9.

Therefore, according to the present preferred embodiment, the apparatus 1 comprises, in sequence relative to the advancement direction, the position sensors 11, if present, the front light source 3, the first line scan cameras 9, the separating partition, if present, and, lying in the same second plane, the second line scan cameras 10 and the back light source 4.

Preferably, each line scan camera 9, 10 has a sensor having a width of 4096 pixels of resolution with a 50 mm objective so as to ensure the discrimination of details of about 1 mm².

As is well known, the viewing angle F of a line scan camera is substantially two-dimensional so that it acquires a single line or linear portion of the framed object. The thickness of that linear portion is closely dependent on the features of the sensor installed in the line scan camera.

Preferably, moreover, each series of first line scan cameras 9 and second line scan cameras 10 is installed at a height of 3 metres and lies substantially in a same horizontal plane. In this manner, each viewing angle F of the line scan cameras is perpendicular to the rest surface 2 and turned towards different parts of the main surface A of the leather hide.

According to one aspect of the invention, the control unit is configured to join each two-dimensional image of the first plurality of two-dimensional images in order to obtain the first image of the leather hide. In addition, the control unit is configured to join each two-dimensional image of the second plurality of two-dimensional images in order to obtain the second image of the leather hide.

As is well known, a line scan camera has a sensor, also known as a CCD or photodiode sensor, made up of a single line of photosensitive elements (or pixels). Therefore, the scan line may be considered as a one-dimensional mapping of the single points of the line of observation. Consequently, this type of camera does not directly provide a photograph of the framed object, but only one line of this fictitious photograph. In order to be able to obtain the actual overall photograph, it is necessary for there to be a relative motion between the object to be scanned and the camera, such as to enable the acquisition, in series, of all the lines necessary to compose the complete photograph.

According to a possible embodiment, the scanning device 5 is configured to acquire the first image and the second image of the leather hide in two different moments. Therefore, for example, in a first condition of movement of the rest surface 2 (and of the leather hide), the scanning device 5 is capable of acquiring the first image of the leather hide, as it is illuminated by the front light source 3. Subsequently, in a second condition of movement, the scanning device 5 is capable of acquiring the second image of the leather hide, as it is illuminated by the back light source 4.

The image acquisition sequence can also be inverted.

Moreover, it is also possible for the first and the second condition of moving near to occur consecutively in an opposite manner or for them to be identical, with a condition of restoring the initial positions of the rest surface 2 relative to the scanning device 5 interposed between them.

The position sensor 11 is capable of detecting the passage of the leather hide before the latter is actually below the image acquisition devices and, therefore, to provide the control unit with the information necessary to define the activation or deactivation of the scanning device 5 and of the light sources 3, 4.

Preferably, the apparatus 1 comprises eight reflective position sensors 11, for example of the optoelectronic type, configured for remote optical detection of objects and arranged above the rest surface 2. Even more preferably, the position sensors 11 are arranged perpendicular to the rest surface 2. In addition, the position sensors 11 are arranged aligned along a same direction transversal to the longitudinal direction L, substantially equidistant from one another by a width equal to about the width of the rest surface 2. Therefore, in this manner each position sensor 11 is configured to equally subdivide the width of the rest surface 2 in order to detect the presence of the leather hide. Preferably, each position sensor 11 is configured to detect the variation of distance that normally exists between them and the rest surface 2. A reduction of this distance is equivalent to the presence of the leather hide, which has a nominal thickness of a few millimetres.

According to one embodiment of the invention, the logic of use of the laser sensors provides for the latter to be activated simultaneously with the start of the movement of the motorised conveyor belt 7. Each pair of first line scan cameras 9 and second line scan cameras 10 begins acquiring the first and second images of the main surface A of the leather hide when at least one of the laser sensors detects the passage of the same leather hide. The end of the scan is instead signalled by the lack of a detection signal from each of the laser sensors.

## Claims

1. A method (100) for identifying and classifying surface defects of a leather hide having an upper surface conventionally called the "grain side" and a lower surface conventionally called the "flesh side", comprising the following steps:
a) placing the leather hide on an elongate rest surface (2) having a longitudinal axis (L);
b) providing, above the rest surface, a first light source (3) capable of illuminating from above a first predetermined portion of the upper surface of the leather hide, as well as a second light source (4) capable of illuminating from below a second predetermined portion of the upper surface of the leather hide, through the thickness of the hide itself, wherein the first predetermined portion and the second predetermined portion of the leather hide are offset from each other along the longitudinal axis (L);
c) illuminating the first predetermined portion of the upper surface of the leather hide (A) with the first light source (3);
d) illuminating the second predetermined portion of the upper surface of the leather hide with the second light source (4);
e) providing a scanning device (5) having a first series of line scan cameras (9) aligned with one another and a second series of line scan cameras (10) aligned with one another and arranged orthogonally relative to the longitudinal axis (L), wherein the first series of line scan cameras (9) is offset from the second series of line scan cameras (10) along the longitudinal axis (L), and wherein the first series of line scan cameras (9) is configured to acquire a first image of the first predetermined portion of the upper surface of the leather hide and the second series of line scan cameras (10) is configured to acquire a second image of said second predetermined portion of the upper surface of the leather hide;
f) acquiring, with the scanning device (5), a first image of the first predetermined portion of the upper surface of the leather hide illuminated by said first light source (3), said first image being representative of a first series of surface characteristics of the leather hide;
g) acquiring, with said scanning device (5), a second image of the second portion of the upper surface of the leather hide illuminated by said second light source (4) through the thickness of the leather hide, said second image being representative of a second series of surface characteristics of the leather hide;
h) moving the leather hide along the longitudinal axis (L) and repeating steps f) and g);
i) comparing, with at least one control unit, said first image and said second image with at least one reference image or model contained in a storage unit in order to identify possible surface defects of the leather hide; the reference image or model being representative of a series of reference surface characteristics of a leather hide with and/or without surface defects; said control unit being connected to said storage unit and to said scanning device (5).

2. The method (100) according to claim 1, wherein said step of comparing with said control unit comprises the following steps:
- receiving at least said first image and said second image from said scanning device (5);
- running a surface pattern analysis software application programmed to compare said first image and said second image with each reference image or model contained in said storage unit;
- detecting possible surface defects present on the leather hide from said first series of surface characteristics of the first image and/or from said second series of surface characteristics of the second image;
- sending to an external display device an output signal representative at least of the presence and/or absence of the possible identified surface defects.

3. The method (100) according to claim 1 or 2, comprising the step of storing said first image and/or said second image in said storage unit.

4. The method (100) according to any preceding claim, comprising the step of joining said first image and said second image in an overall image representative both of said first series of surface characteristics and of said second series of surface characteristics of the leather hide.

5. The method (100) according to one of the preceding claims, comprising a step of synchronising the reciprocal movement between said rest surface (2) and said scanning device (5) based on a value of frequency of acquisition of said first image and/or said second image of said scanning device (5).

6. The method (100) according to any one of the claims 1 to 5, wherein said step of acquiring said first image and said step of acquiring said second image are carried out simultaneously.

7. The method (100) according to any preceding claim, wherein said steps of illuminating with said first light source (3), illuminating with said second light source (4), acquiring said first image and acquiring said second image are carried out by said control unit on the basis of a position signal received from at least one position sensor (11), said position sensor (11) being configured to detect the presence of the leather hide at the start of the first and/or second portion of the upper surface of the leather hide, as defined by a viewing angle (F) of the line scan cameras (9, 10) of the scanning device (5).

8. An apparatus (1) for identifying and classifying surface defects of a leather hide, comprising:
- an elongate rest surface (2) having a longitudinal axis (L) on which a leather hide can be positioned and moved in the direction of the longitudinal axis (L);
- a first light source (3) configured to illuminate from above a first predetermined portion of an upper surface of the leather hide;
- a second light source (4) configured to illuminate from below a second predetermined portion, offset (L) from the first predetermined portion of the upper surface of the leather hide along the longitudinal axis; **characterised in that** said apparatus further comprises:
- a scanning device (5) having a first series of line scan cameras (9) aligned with one another and a second series of line scan cameras (10) aligned with one another, said first and second series of line scan cameras (9, 10) being arranged respectively aligned and on planes orthogonal to the longitudinal axis (L), and wherein the second series of line scan cameras (10) is arranged offset from the first series of line scan cameras (9) along the longitudinal axis (L), and wherein each series of cameras (9, 10) defines a viewing angle (F) such as to frame a portion of said rest surface (2); the first series of line scan cameras (9) being configured to acquire a first image of the first predetermined portion of the upper surface of the leather hide illuminated from above by said first light source (3) and the second series of line scan cameras (10) being configured to acquire a second image of the second portion of the upper surface of the leather hide illuminated from below by said second light source (4);
- a control unit connected to at least one said scanning device (5) and programmed to identify the presence of possible surface defects on each image acquired; said control unit being configured to implement a method for identifying and classifying surface defects of a leather hide according to any one of claims 1 to 7.

9. The apparatus (1) according to claim 8, **characterised in that** said rest surface (2) comprises a motorised conveyor belt (7) extending along the longitudinal axis (L) and configured to convey the leather hide forward along said longitudinal axis (L).

10. The apparatus (1) according to claim 9 **characterised in that** the first series of line scan cameras (9) is configured to acquire a first series of two-dimensional images relating to said first portion of the leather hide during the movement of the leather hide on said motorised conveyor belt.

11. The apparatus (1) according to claim 10, **characterised in that** said second series of line scan cameras (10) is configured to acquire a second series of two-dimensional images relating to said second portion of the leather hide during the movement of the leather hide on said motorised conveyor belt.

12. The apparatus (1) according to claim 10 and/or 11, **characterised in that** said control unit is configured to join together each two-dimensional image of said first plurality of two-dimensional images to obtain said first image of the leather hide; said control unit being further configured to join each two-dimensional image of said second plurality of two-dimensional images to obtain said second image of the leather hide.

13. The apparatus (1) according to any one of the preceding claims 9 to 13, **characterised in that** it further comprises a synchronisation device associated with said motorised conveyor belt and with said scanning device (5) and configured to synchronise the advancement of the leather hide on the motorised conveyor belt based on a value of frequency of acquisition of the images of said scanning device (5).

14. The apparatus (1) according to any one of the preceding claims 9 to 13, **characterised in that** it further comprises at least one position sensor (11) connected to said control unit and configured to detect the presence or absence of a leather hide in a portion of said rest surface (2) framed by said scanning device (5); said control unit being configured to activate said scanning device (5), said first light source (3) and said second light source (4) on the basis of the detection implemented by said at least one position sensor (11).

## Patentansprüche

1. Verfahren (100) zur Erkennung und Klassifizierung von Oberflächenfehlern einer Lederhaut mit einer oberen Oberfläche, die üblicherweise als "Narbenseite" bezeichnet wird, und einer unteren Oberfläche, die üblicherweise als "Fleischseite" bezeichnet wird, das die folgenden Schritte umfasst:
a) Anordnen der Lederhaut auf einer länglichen Auflageoberfläche (2) mit einer Längsachse (L);
b) Bereitstellen, über der Auflageoberfläche, einer ersten Lichtquelle (3), die in der Lage ist, einen ersten vorbestimmten Abschnitt der oberen Oberfläche der Lederhaut von oben zu beleuchten, sowie einer zweiten Lichtquelle (4), die in der Lage ist, einen zweiten vorbestimmten Abschnitt der oberen Oberfläche der Lederhaut von unten durch die Dicke der Haut selbst zu beleuchten, wobei der erste vorbestimmte Abschnitt und der zweite vorbestimmte Abschnitt der Lederhaut entlang der Längsachse (L) voneinander versetzt sind;
c) Beleuchten des ersten vorbestimmten Abschnitts der oberen Oberfläche der Lederhaut (A) mit der ersten Lichtquelle (3);
d) Beleuchten des zweiten vorbestimmten Abschnitts der oberen Oberfläche der Lederhaut mit der zweiten Lichtquelle (4);
e) Bereitstellen einer Abtastvorrichtung (5), die eine erste Reihe von Zeilenabtastkameras (9), die zueinander ausgerichtet sind, und eine zweite Reihe von Zeilenabtastkameras (10), die zueinander ausgerichtet und orthogonal relativ zu der Längsachse (L) angeordnet sind, aufweist, wobei die erste Reihe von Zeilenabtastkameras (9) von der zweiten Reihe von Zeilenabtastkameras (10) entlang der Längsachse (L) versetzt ist, und wobei die erste Reihe von Zeilenabtastkameras (9) ausgelegt ist, um ein erstes Bild des ersten vorbestimmten Abschnitts der oberen Oberfläche der Lederhaut zu erfassen, und die zweite Reihe von Zeilenabtastkameras (10) ausgelegt ist, um ein zweites Bild des zweiten vorbestimmten Abschnitts der oberen Oberfläche der Lederhaut zu erfassen;
f) Erfassen, mit der Abtastvorrichtung (5), eines ersten Bildes des ersten vorbestimmten Abschnitts der oberen Oberfläche der Lederhaut, die von der ersten Lichtquelle (3) beleuchtet wird, wobei das erste Bild für eine erste Reihe von Oberflächenmerkmalen der Lederhaut repräsentativ ist;
g) Erfassen, mit der Abtastvorrichtung (5), eines zweiten Bildes des zweiten Abschnitts der oberen Oberfläche der Lederhaut, die von der zweiten Lichtquelle (4) durch die Dicke der Lederhaut beleuchtet wird, wobei das zweite Bild für eine zweite Reihe von Oberflächenmerkmalen der Lederhaut repräsentativ ist;
h) Bewegen der Lederhaut entlang der Längsachse (L) und Wiederholen der Schritte f) und g);
i) Vergleichen des ersten Bildes und des zweiten Bildes mit mindestens einem in einer Speichereinheit enthaltenen Referenzbild oder -modell mit mindestens einer Steuereinheit, um mögliche Oberflächenfehler der Lederhaut zu erkennen; wobei das Referenzbild oder - modell für eine Reihe von Referenzoberflächenmerkmalen einer Lederhaut mit und/oder ohne Oberflächenfehler repräsentativ ist; wobei die Steuereinheit mit der Speichereinheit und der Abtastvorrichtung (5) verbunden ist.

2. Verfahren (100) nach Anspruch 1, wobei der Schritt zum Vergleichen mit der Steuereinheit die folgenden Schritte umfasst:
- Empfangen mindestens des ersten Bildes und des zweiten Bildes von der Abtastvorrichtung (5);
- Ausführen einer Oberflächenmusteranalysesoftwareanwendung, die programmiert ist, um das erste Bild und das zweite Bild mit einem jeden Referenzbild oder -modell zu vergleichen, das in der Speichereinheit enthalten ist;
- Detektieren möglicher Oberflächenfehler, die auf der Lederhaut vorhanden sind, aus der ersten Reihe von Oberflächenmerkmalen des ersten Bildes und/oder aus der zweiten Reihe von Oberflächenmerkmalen des zweiten Bildes;
- Senden eines Ausgangssignals, das mindestens für das Vorhandensein und/oder Fehlen der möglichen erkannten Oberflächenfehler repräsentativ ist, an eine externe Anzeigevorrichtung.

3. Verfahren (100) nach Anspruch 1 oder 2, umfassend den Schritt zum Speichern des ersten Bildes und/oder des zweiten Bildes in der Speichereinheit.

4. Verfahren (100) nach einem der vorhergehenden Ansprüche, umfassend den Schritt zum Zusammenfügen des ersten Bildes und des zweiten Bildes in einem Gesamtbild, das sowohl für die erste Reihe von Oberflächenmerkmalen als auch für die zweite Reihe von Oberflächenmerkmalen der Lederhaut repräsentativ ist.

5. Verfahren (100) nach einem der vorhergehenden Ansprüche, umfassend einen Schritt zum Synchronisieren der Hin- und Herbewegung zwischen der Auflageoberfläche (2) und der Abtastvorrichtung (5) basierend auf einem Wert der Häufigkeit der Erfassung des ersten Bildes und/oder des zweiten Bildes der Abtastvorrichtung (5).

6. Verfahren (100) nach einem der Ansprüche 1 bis 5, wobei der Schritt zum Erfassen des ersten Bildes und der Schritt zum Erfassen des zweiten Bildes gleichzeitig ausgeführt werden.

7. Verfahren (100) nach einem der vorhergehenden Ansprüche, wobei die Schritte zum Beleuchten mit der ersten Lichtquelle (3), zum Beleuchten mit der zweiten Lichtquelle (4), zum Erfassen des ersten Bildes und zum Erfassen des zweiten Bildes durch die Steuereinheit auf der Grundlage eines Positionssignals ausgeführt werden, das von mindestens einem Positionssensor (11) empfangen wird, wobei der Positionssensor (11) ausgelegt ist, um das Vorhandensein der Lederhaut am Anfang des ersten und/oder zweiten Abschnitts der oberen Oberfläche der Lederhaut zu detektieren, wie durch einen Betrachtungswinkel (F) der Zeilenabtastkameras (9, 10) der Abtastvorrichtung (5) definiert ist.

8. Vorrichtung (1) zur Erkennung und Klassifizierung von Oberflächenfehlern einer Lederhaut, umfassend:
- eine längliche Auflageoberfläche (2) mit einer Längsachse (L), auf der eine Lederhaut positioniert und in Richtung der Längsachse (L) bewegt werden kann;
- eine erste Lichtquelle (3), die dazu ausgelegt ist, einen ersten vorbestimmten Abschnitt einer oberen Oberfläche der Lederhaut von oben zu beleuchten;
- eine zweite Lichtquelle (4), die dazu ausgelegt ist, einen zweiten vorbestimmten Abschnitt von unten zu beleuchten, der von dem ersten vorbestimmten Abschnitt der oberen Oberfläche der Lederhaut entlang der Längsachse (L)versetzt ist;
**dadurch gekennzeichnet, dass** die Vorrichtung ferner Folgendes umfasst:
- eine Abtastvorrichtung (5), die eine erste Reihe von Zeilenabtastkameras (9), die zueinander ausgerichtet sind, und eine zweite Reihe von Zeilenabtastkameras (10), die zueinander ausgerichtet sind, aufweist, wobei die erste und die zweite Reihe von Zeilenabtastkameras (9, 10) jeweils ausgerichtet und auf Ebenen orthogonal zur Längsachse (L) angeordnet sind, und wobei die zweite Reihe von Zeilenabtastkameras (10) versetzt von der ersten Reihe von Zeilenabtastkameras (09) entlang der Längsachse (L) angeordnet ist, und wobei eine jede Reihe von Kameras (9, 10) einen Betrachtungswinkel (F) definiert, um einen Abschnitt der Auflageoberfläche (2) einzurahmen;
wobei die erste Reihe von Zeilenabtastkameras (9) dazu ausgelegt ist, ein erstes Bild des ersten vorbestimmten Abschnitts der oberen Oberfläche der Lederhaut, die durch die erste Lichtquelle (3) von oben beleuchtet wird, zu erfassen, und die zweite Reihe von Zeilenkameras (10) dazu ausgelegt ist, ein zweites Bild des zweiten Abschnitts der oberen Oberfläche der Lederhaut, die durch die zweite Lichtquelle (4) von unten beleuchtet wird, zu erfassen;
- eine Steuereinheit, die mindestens mit der Abtastvorrichtung (5) verbunden und programmiert ist, um das Vorhandensein möglicher Oberflächenfehler auf einem jeden erfassten Bild zu erkennen; wobei die Steuereinheit ausgelegt ist, um ein Verfahren zur Erkennung und Klassifizierung von Oberflächenfehlern einer Lederhaut nach einem der Ansprüche 1 bis 7 zu implementieren.

9. Vorrichtung (1) nach Anspruch 8, **dadurch gekennzeichnet, dass** die Auflageoberfläche (2) ein motorisiertes Förderband (7) umfasst, das sich entlang der Längsachse (L) erstreckt und dazu ausgelegt ist, die Lederhaut entlang der Längsachse (L) nach vorne zu befördern.

10. Vorrichtung (1) nach Anspruch 9, **dadurch gekennzeichnet, dass** die erste Reihe von Zeilenabtastkameras (9) ausgelegt ist, um eine erste Reihe von zweidimensionalen Bildern in Bezug auf den ersten Abschnitt der Lederhaut während der Bewegung der Lederhaut auf dem motorisierten Förderband zu erfassen.

11. Vorrichtung (1) nach Anspruch 10, **dadurch gekennzeichnet, dass** die zweite Reihe von Zeilenabtastkameras (10) ausgelegt ist, um eine zweite Reihe von zweidimensionalen Bildern in Bezug auf den zweiten Abschnitt der Lederhaut während der Bewegung der Lederhaut auf dem motorisierten Förderband zu erfassen.

12. Vorrichtung (1) nach Anspruch 10 und/oder 11, **dadurch gekennzeichnet, dass** die Steuereinheit dazu ausgelegt ist, ein jedes zweidimensionale Bild der ersten Vielzahl von zweidimensionalen Bildern miteinander zusammenzufügen, um das erste Bild der Lederhaut zu erhalten; wobei die Steuereinheit ferner dazu ausgelegt ist, ein jedes zweidimensionale Bild der zweiten Vielzahl von zweidimensionalen Bildern zusammenzufügen, um das zweite Bild der Lederhaut zu erhalten.

13. Vorrichtung (1) nach einem der vorhergehenden Ansprüche 9 bis 13, **dadurch gekennzeichnet, dass** sie ferner eine Synchronisationsvorrichtung umfasst, die mit dem motorisierten Förderband und der Abtastvorrichtung (5) assoziiert ist und ausgelegt ist, um den Vorschub der Lederhaut auf dem motorisierten Förderband auf Grundlage eines Werts der Häufigkeit der Erfassung der Bilder der Abtastvorrichtung (5) zu synchronisieren.

14. Vorrichtung (1} nach einem der vorhergehenden Ansprüche 9 bis 13, **dadurch gekennzeichnet, dass** sie ferner mindestens einen Positionssensor (11) umfasst, der mit der Steuereinheit verbunden und ausgelegt ist, um das Vorhandensein oder Fehlen einer Lederhaut in einem Abschnitt der Auflageoberfläche (2) zu detektieren, der von der Abtastvorrichtung (5) umrahmt wird; wobei die Steuereinheit ausgelegt ist, um die Abtastvorrichtung (5), die erste Lichtquelle (3) und die zweite Lichtquelle (4) auf der Grundlage der Detektion zu aktivieren, die von dem mindestens einen Positionssensor (11) implementiert wird.

## Revendications

1. Procédé (100) pour identifier et classer des défauts de surface d'une peau de cuir ayant une surface supérieure conventionnellement appelée « côté fleur » et une surface inférieure conventionnellement appelée « côté chair », comprenant les étapes suivantes :
a) placer la peau de cuir sur une surface d'appui allongée (2) ayant un axe longitudinal (L) ;
b) fournir, au-dessus de la surface d'appui, une première source lumineuse (3) capable d'éclairer par le haut une première partie prédéterminée de la surface supérieure de la peau de cuir, ainsi qu'une seconde source lumineuse (4) capable d'éclairer par le bas une seconde partie prédéterminée de la surface supérieure de la peau de cuir, à travers l'épaisseur de la peau elle-même, dans lequel la première partie prédéterminée et la seconde partie prédéterminée de la peau de cuir sont décalées l'une par rapport à l'autre le long de l'axe longitudinal (L) ;
c) éclairer la première partie prédéterminée de la surface supérieure de la peau de cuir (A) avec la première source lumineuse (3) ;
d) éclairer la seconde partie prédéterminée de la surface supérieure de la peau de cuir avec la seconde source lumineuse (4) ;
e) fournir un dispositif de balayage (5) comportant une première série de caméras à balayage linéaire (9) alignées les unes avec les autres et une seconde série de caméras à balayage linéaire (10) alignées les unes avec les autres et disposées orthogonalement par rapport à l'axe longitudinal (L), dans lequel la première série de caméras à balayage linéaire (9) est décalée par rapport à la seconde série de caméras à balayage linéaire (10) le long de l'axe longitudinal (L), et dans lequel la première série de caméras à balayage linéaire (9) est configurée pour acquérir une première image de la première partie prédéterminée de la surface supérieure de la peau de cuir et la seconde série de caméras à balayage linéaire (10) est configurée pour acquérir une seconde image de ladite seconde partie prédéterminée de la surface supérieure de la peau de cuir ;
f) acquérir, à l'aide du dispositif de balayage (5), une première image de la première partie prédéterminée de la surface supérieure de la peau de cuir éclairée par ladite première source lumineuse (3), ladite première image étant représentative d'une première série de caractéristiques de surface de la peau de cuir ;
g) acquérir, à l'aide dudit dispositif de balayage (5), une seconde image de la seconde partie de la surface supérieure de la peau de cuir éclairée par ladite seconde source lumineuse (4) à travers l'épaisseur de la peau de cuir, ladite seconde image étant représentative d'une seconde série de caractéristiques de surface de la peau de cuir ;
h) déplacer la peau de cuir le long de l'axe longitudinal (L) et répéter les étapes f) et g) ;
i) comparer, à l'aide d'au moins une unité de contrôle, ladite première image et ladite seconde image avec au moins une image ou un modèle de référence contenu dans une unité de stockage afin d'identifier d'éventuels défauts de surface de la peau de cuir ; l'image ou le modèle de référence étant représentatif d'une série de caractéristiques de surface de référence d'une peau de cuir avec et/ou sans défaut(s) de surface ; ladite unité de contrôle étant reliée à ladite unité de stockage et audit dispositif de balayage (5).

2. Procédé (100) selon la revendication 1, dans lequel ladite étape de comparaison avec ladite unité de contrôle comprend les étapes suivantes :
- recevoir au moins ladite première image et ladite seconde image provenant dudit dispositif de balayage (5) ;
- exécuter une application logicielle d'analyse de motifs de surface programmée pour comparer ladite première image et ladite seconde image avec chaque image ou modèle de référence contenu dans ladite unité de stockage ;
- détecter les défauts de surface possibles présents sur la peau de cuir à partir de ladite première série de caractéristiques de surface de la première image et/ou à partir de ladite seconde série de caractéristiques de surface de la seconde image ;
- envoyer à un dispositif d'affichage externe un signal de sortie représentatif au moins de la présence et/ou de l'absence des défauts de surface possibles identifiés.

3. Procédé (100) selon la revendication 1 ou 2, comprenant l'étape consistant à stocker ladite première image et/ou ladite seconde image dans ladite unité de stockage.

4. Procédé (100) selon l'une quelconque des revendications précédentes, comprenant l'étape consistant à assembler ladite première image et ladite seconde image en une image globale représentative à la fois de ladite première série de caractéristiques de surface et de ladite seconde série de caractéristiques de surface de la peau de cuir.

5. Procédé (100) selon l'une des revendications précédentes, comprenant une étape consistant à synchroniser le mouvement réciproque entre ladite surface d'appui (2) et ledit dispositif de balayage (5) sur la base d'une valeur de fréquence d'acquisition de ladite première image et/ou de ladite seconde image dudit dispositif de balayage (5).

6. Procédé (100) selon l'une quelconque des revendications 1 à 5, dans lequel ladite étape d'acquisition de ladite première image et ladite étape d'acquisition de ladite seconde image sont effectuées simultanément.

7. Procédé (100) selon l'une quelconque des revendications précédentes, dans lequel lesdites étapes consistant à éclairer avec ladite première source lumineuse (3), à éclairer avec ladite seconde source lumineuse (4), à acquérir ladite première image et à acquérir ladite seconde image sont effectuées par ladite unité de contrôle sur la base d'un signal de position reçu d'au moins un capteur de position (11), ledit capteur de position (11) étant configuré pour détecter la présence de la peau de cuir au début de la première et/ou de la seconde partie de la surface supérieure de la peau de cuir, telle que définie par un angle de visualisation (F) des caméras à balayage linéaire (9, 10) du dispositif de balayage (5).

8. Appareil (1) identifiant et classant les défauts de surface d'une peau de cuir, comprenant :
- une surface d'appui allongée (2) ayant un axe longitudinal (L) sur laquelle une peau de cuir peut être positionnée et déplacée dans la direction de l'axe longitudinal (L) ;
- une première source lumineuse (3) configurée pour éclairer par le haut une première partie prédéterminée d'une surface supérieure de la peau de cuir ;
- une seconde source lumineuse (4) configurée pour éclairer par le bas une seconde partie prédéterminée, décalée par rapport à la première partie prédéterminée de la surface supérieure de la peau de cuir le long de l'axe longitudinal (L) ;
**caractérisé en ce que** ledit appareil comprend en outre :
- un dispositif de balayage (5) comportant une première série de caméras à balayage linéaire (9) alignées les unes avec les autres et une seconde série de caméras à balayage linéaire (10) alignées les unes avec les autres, lesdites première et seconde séries de caméras à balayage linéaire (9, 10) étant disposées respectivement de façon alignée et sur des plans orthogonaux à l'axe longitudinal (L), et dans lequel la seconde série de caméras à balayage linéaire (10) est disposée de façon décalée par rapport à la première série de caméras à balayage linéaire (9) le long de l'axe longitudinal (L), et dans lequel chaque série de caméras (9, 10) définit un angle de visualisation (F) de manière à encadrer une partie de ladite surface d'appui (2) ;
la première série de caméras à balayage linéaire (9) étant configurée pour acquérir une première image de la première partie prédéterminée de la surface supérieure de la peau de cuir éclairée par le haut par ladite première source lumineuse (3) et la seconde série de caméras à balayage linéaire (10) étant configurée pour acquérir une seconde image de la seconde partie de la surface supérieure de la peau de cuir éclairée par le bas par ladite seconde source lumineuse (4) ;
- une unité de contrôle reliée au moins au dispositif de balayage (5) et programmée pour identifier la présence d'éventuels défauts de surface sur chaque image acquise ; ladite unité de contrôle étant configurée pour mettre en œuvre un procédé d'identification et de classement des défauts de surface d'une peau de cuir selon l'une quelconque des revendications 1 à 7.

9. Appareil (1) selon la revendication 8, **caractérisé en ce que** ladite surface d'appui (2) comprend une bande transporteuse motorisée (7) s'étendant le long de l'axe longitudinal (L) et configurée pour transporter la peau de cuir vers l'avant le long dudit axe longitudinal (L).

10. Appareil (1) selon la revendication 9, **caractérisé en ce que** la première série de caméras à balayage linéaire (9) est configurée pour acquérir une première série d'images bidimensionnelles relatives à ladite première partie de la peau de cuir pendant le mouvement de la peau de cuir sur ladite bande transporteuse motorisée.

11. Appareil (1) selon la revendication 10, **caractérisé en ce que** ladite seconde série de caméras à balayage linéaire (10) est configurée pour acquérir une seconde série d'images bidimensionnelles relatives à ladite seconde partie de la peau de cuir pendant le mouvement de la peau de cuir sur ladite bande transporteuse motorisée.

12. Appareil (1) selon la revendication 10 et/ou 11, **caractérisé en ce que** ladite unité de contrôle est configurée pour assembler chaque image bidimensionnelle de ladite première pluralité d'images bidimensionnelles afin d'obtenir ladite première image de la peau de cuir ; ladite unité de contrôle étant en outre configurée pour assembler chaque image bidimensionnelle de ladite seconde pluralité d'images bidimensionnelles afin d'obtenir ladite seconde image de la peau de cuir.

13. Appareil (1) selon l'une quelconque des revendications précédentes 9 à 13, **caractérisé en ce qu'**il comprend en outre un dispositif de synchronisation associé à ladite bande transporteuse motorisée et audit dispositif de balayage (5) et configuré pour synchroniser l'avancement de la peau de cuir sur la bande transporteuse motorisée sur la base d'une valeur de fréquence d'acquisition des images dudit dispositif de balayage (5).

14. Appareil (1) selon l'une quelconque des revendications précédentes 9 à 13, **caractérisé en ce qu'**il comprend en outre au moins un capteur de position (11) relié à ladite unité de contrôle et configuré pour détecter la présence ou l'absence d'une peau de cuir dans une partie de ladite surface d'appui (2) encadrée par ledit dispositif de balayage (5) ; ladite unité de contrôle étant configurée pour activer ledit dispositif de balayage (5), ladite première source lumineuse (3) et ladite seconde source lumineuse (4) sur la base de la détection mise en œuvre par ledit au moins un capteur de position (11).
